# EUROPÄISCHE PATENTANMELDUNG

(11) **EP 1 034 762 A2**
(43) Veröffentlichungstag der Anmeldung: **13.09.2000**
(21) Anmeldenummer: 00101836.5
(22) Anmeldetag: 29.01.2000
(51) Int. Cl.: A61F 13/551, A61F 15/00

(54) **Verpackungshülle**

(30) Priorität: 06.03.1999 DE 19909839
(71) Anmelder: 4P Folie Forchheim GmbH, 91301 Forchheim (DE)
(72) Erfinder: Schmidt, Werner, 96129 Strullendorf (DE); Ihde, Thomas, 91301 Forchheim (DE)
(74) Vertreter: Hutzelmann, Gerhard

(57) **Zusammenfassung**

Verpackungshülle, insbesondere zum Verpackung von Damenbinden, mit einer Antihaftbeschichtung auf der Innenseite der Verpackungshülle, wobei die Außenseite der Verpackungshülle eine textilähnliche Aufmachung aufweist.

## Beschreibung

Die Erfindung betrifft eine Verpackungshülle, insbesondere zum Verpackung von Damenbinden, mit einer Antihaftbeschichtung auf der Innenseite der Verpackungshülle.

Der Erfindung liegt die Aufgabe zugrunde, eine Verpackungshülle der genannten so auszugestalten, daß sie den geforderten hygenischen Anforderungen entspricht und darüber hinaus einen komfortablen Eindruck macht.

Diese Aufgabe wird erfindungsgemäß dadurch gelöst, daß die Außenseite der Verpackungshülle eine textilähnliche Aufmachung aufweist.

Eine vorteilhafte Ausgestaltung der Erfindung liegt darin, daß die Verpackungshülle von einer Kunststoffolie gebildet ist, deren Außenseite eine Prägung aufweist, die ihr ein textilähnliches Aussehen verleiht.

Vorteilhaft ist es dabei auch, wenn die Verpackungshülle eine äußere geprägte und eine innere glatte Kunststoffolie aufweist.

Eine weitere vorteilhafte Ausgestaltung der Erfindung liegt darin, daß die Verpackungshülle von einem Vliesstoff gebildet ist.

Als sehr vorteilhaft hat es sich auch erwiesen, wenn gemäß einer weiteren Ausgestaltung der Erfindung der Vliesstoff vorzugsweise an seiner Innenseite mit einer Kunststoffolie verbunden ist.

Durch alle diese Ausgestaltungen wird eine komfortable Außenseite der Verpackungshülle geschaffen, die entsprechend den jeweiligen Ansprüchen an Dichtigkeit ausgestaltet werden kann.

Eine weitere vorteilhafte Ausgestaltung der Erfindung ist dadurch gekennzeichnet, daß die Antihaftbeschichtung auf der Innenseite der Verpackungshülle abschnittsweise vorgesehen ist.

Dadurch ist es möglich, einseitig klebende Damenbinden in die Verpackungshülle einzulegen, die wieder leicht entnehmbar sind. Gleichzeitig ist es aber möglich, die nicht mit der Beschichtung versehenen Abschnitte zum Bilden der Verpackungshülle miteinander zu versiegeln oder zu verkrümpern.

In der Zeichnung ist die Erfindung anhand von drei Ausführungsbeispielen veranschaulicht. Dabei zeigen:
- Fig. 1: einen Vertikalschnitt durch einen Materialabschnitt, mit einer Kunststoffolie, die einseitig mit einem Vlies und auf der anderen Seite mit einer partiell aufgetragenen Antihaftbeschichtung versehen ist,
- Fig.2: einen Vertikalschnitt durch einen weiteren Materialabschnitt, der einschichtig aus einem Vlies gebildet ist, das auf der späteren Innenseite der Verpackungshülle mit einer partiellen Antihaftbeschichtung versehen ist und
- Fig.3: einen Vertikalschnitt durch einen Materialabschnitt, der aus einer geprägten Kunststoffolie besteht.

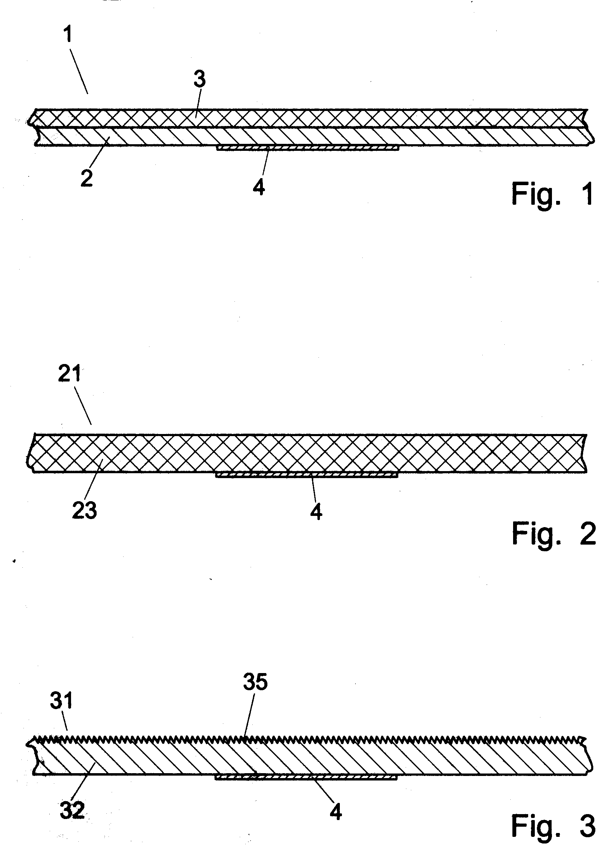

Mit 1 ist in Fig. 1 ein Materialabschnitt bezeichnet, der zum Herstellen einer Verpackungshülle vorgesehen ist, die ihrerseits zum Verpacken von Damenbinden dienen soll. Der Materialabschnitt 1 besteht aus einer Kunststoffolie 2, auf deren einer Seite ein Vlies 3 aufgebracht ist. Auf der vom Vlies abgewandten Seite ist die Kunststoffolie mit einer Anthaftbeschichtung 4 versehen, die nur partiell aufgebracht ist. Beim Zusammenfalten des Materialabschnittes 1 kommt die Antihaftbeschichtung 4 nach innen und ermöglicht ein nur leichtes Ankleben der mit einer klebenden Seite ausgerüsteten Damenbinde. Durch die Faltung des Materialabschnittes 1 ergeben sich auf der Innenseite der Verpackung Bereiche, in denen wenigstens zwei Lagen der Kunststoffolie 2 direkt aufeinander zu liegen kommen. In diesen Bereichen werden die Lagen der Kunststoffolie 2 miteinander versiegelt oder verkrümpert. Das Versiegeln oder Verkrümpern wird durch die Antihaftbeschichtung 4 nicht beeinträchtigt. Die nur leicht an der Antihaftbeschichtung 4 anhaftende Damenbinde läßt sich leicht aus der Verpackung entnehmen.

Der Materialabschnitt 21 besteht aus einem Vlies 23. Dieses Vlies 23 ist auf seiner einen Seite partiell mit einer Antihaftbeschichtung 4 versehen. Durch Zusammenfalten des Materialabschnittes kommt die Antihaftbeschichtung 4 ebenso wie beim oben angeführten Ausführungsbeispiel nach innen. Die klebende Seite einer Damenbinde haftet nur leicht an der Antihaftbeschichtung 4 an. Eine leichte Entnehmbarkeit der Damenbinde aus der Verpackung ist somit gewährleistet. Ebenso ist durch die nur partielle Antihaftbeschichtung 4 die Siegel- und Krümperfähigkeit der zusammengefalteten Vlies-Lagen sichergestellt.

Das in Fig. 3 dargestellte weitere Ausführungsbeispiel zeigt einen Materialabschnitt 31. Dieser Materialabschnitt 31 besteht aus einer Kunststoffolie 32, auf deren einer Seite eine textilähnliche Prägung 35 angeordnet ist. Auf der der Prägung 35 abgewandten Seite ist die Folie 32 mit einer partiell aufgebrachten Antihaftbeschichtung 4 versehen. Die Antihaftbeschichtung 4 kommt beim Zusammenfalten des Materialabschnittes 31 nach innen. Durch die nur partiell aufgebrachte Antihaftbeschichtung 4 ist in den von der Antihaftbeschichtung 4 ausgesparten Bereichen eine Aneinandersiegelung oder Verkrümperung der sich beim Zusammenfalten bildenden mehreren Lagen des Materialabschnittes 31 ohne Probleme möglich. Die durch die Verpackung umschlossenen Damenbinde läßt sich aufgrund des nur leichten Anhaftens ihrer Klebeseite an der Antihaftbeschichtung 4 leicht aus der Verpackung herausnehmen.

## Patentansprüche

1. Verpackungshülle, insbesondere zum Verpackung von Damenbinden, mit einer Antihaftbeschichtung auf der Innenseite der Verpackungshülle, **dadurch gekennzeichnet**, daß die Außenseite der Verpackungshülle eine textilähnliche Aufmachung aufweist.

2. Verpackungshülle nach Anspruch 1, **dadurch gekennzeichnet**, daß die Verpackungshülle von einer Kunststoffolie gebildet ist, deren Außenseite eine Prägung aufweist, die ihr ein textilähnliches Aussehen verleiht.

3. Verpackungshülle nach Anspruch 2, **dadurch gekennzeichnet**, daß die Verpackungshülle eine äußere geprägte und eine innere glatten Kunststoffolie aufweist.

4. Verpackungshülle nach Anspruch 1, **dadurch gekennzeichnet**, daß die Verpackungshülle von einem Vliesstoff gebildet ist.

5. Verpackungshülle nach Anspruch 4, **dadurch gekennzeichnet**, daß der Vliesstoff vorzugsweise an seiner Innenseite mit einer Kunststoffolie verbunden ist.

6. Verpackung nach einem der vorhergehenden Ansprüche, **dadurch gekennzeichnet**, daß die Antihaftbeschichtung auf der Innenseite der Verpackungshülle abschnittsweise vorgesehen ist.
